(19) Europäisches Patentamt
European Patent Office
Office européen des brevets

(11) Publication number: 0 008 936
B1

(12) # EUROPEAN PATENT SPECIFICATION

(45) Date of publication of patent specification: 16.02.83

(51) Int. Cl.³: C 07 F 15/00 //C07C87/36

(21) Application number: 79301803.7

(22) Date of filing: 31.08.79

(54) **Platinum complexes, processes for their preparation, pharmaceutical compositions containing them and their use as medicaments.**

(30) Priority: 02.09.78 JP 107776/78

(43) Date of publication of application: 19.03.80 Bulletin 80/6

(45) Publication of the grant of the patent: 16.02.83 Bulletin 83/7

(84) Designated Contracting States: DE FR GB IT

(56) References cited:
FR - A - 2 187 345
US - A - 4 115 418

CHEMICAL ABSTRACTS, vol. 84, no. 9, 1st March 1976, page 44, no. 54030n Columbus, Ohio, U.S.A. R. J. SPEER et al.: "Malonato-1,2-diaminocyclohexaneplatinum(II), a potential antitumor agent"

The file contains technical information submitted after the application was filed and not included in this specification

(73) Proprietor: KIDANI, YOSHINORI
2-718, Mataho Kodan-jutaku
2-1, Mataho-cho Nishi-ku, Nagoya-shi (JP)

(72) Inventor: Kidani, Yoshinori
2-718, Mataho Kodan-jutaku
2-1, Mataho-cho Nishi-ku, Nagoya-shi (JP)
Inventor: Okamoto, Koji
66, Higashi-yashiki
Oshikamo-cho Toyota-shi, Aichi-ken (JP)
Inventor: Saito, Reiko
1-10-12, Futamura-dai
Toyoake-shi, Aichi-ken (JP)

(74) Representative: Woodman, Derek et al,
Frank B. Dehn & Co. European Patent Attorneys
Imperial House 15-19 Kingsway
London WC2B 6UZ (GB)

EP 0 008 936 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

(56) References cited:
CHEMICAL ABSTRACTS, vol. 87, no. 19, 7th November 1977, page 14, no. 145547q Columbus, Ohio, U.S.A. Z. SIMON et al.: "Quantitative structure — antitumor activity relations for complex platinum(II) compounds with amine ligands"

CHEMICAL ABSTRACTS, Chemical Substance Index, vol. 87, July-December 1977, page 1766, Columbus, Ohio, U.S.A.

Platinum complexes, processes for their preparation, pharmaceutical compositions containing them and their use as medicaments

This invention relates to platinum complexes, processes for their preparation, pharmaceutical compositions containing them and their use as medicaments.

It is known that certain platinum complexes are active against tumour cells [for example, Nature, *222*, 385 (1969); Platinum Metal Rev., *15*, No. 2, 42—51 (1971); ibid. *17*, No. 1, 2—13 (1973) and US Patent 4,115,418 (1978)].

In particular cis dichlorodiammineplatinium (II) has been found to possess antitumour activity, but as a result of its toxic side effects has a rather low therapeutic index. In Wadley Med. Bull. 1975, 5(4), 335—348 malonato 1,2-diaminocyclohexaneplatinum (II) is described, the cis and trans (1) isomers of which possess a therapeutic index twice as high as that of cis dichlorodiammineplatinum (II).

The present invention is based on the discovery that certain 1-aminomethyl-2-aminocyclohexane platinum complexes exhibit good anti-tumour activity and low toxicity and thus possess a relatively high therapeutic index. Compounds of the present invention which have been tested have been found to possess a therapeutic index at least as high as and generally higher than the cis or trans (1) isomer of malonato 1,2-diaminocyclohexaneplatinum (II). In fact certain compounds of the present invention possess a therapeutic index eight times greater than the therapeutic index of the aforesaid cis and trans (1) isomers.

Thus, the present invention provides a platinum complex of the general formula:

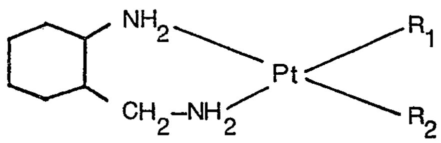

I

(wherein

a) $R_1$ and $R_2$ are the same and each represent a chlorine, bromine or iodine atom or $NO_3$;

or

b) $R_1$ is $SO_4$ and $R_2$ is $H_2O$;

or

c) $R_1$ and $R_2$ together represent:—

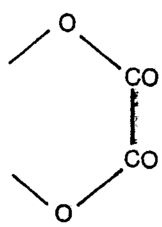 , 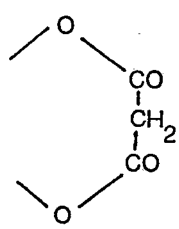

or a glucuronate moiety), the configuration of the 1-aminomethyl-2-aminocyclohexane being selected from *trans*-l, *trans*-d, *cis*-l and *cis*-d, with the proviso that $R_1$ and $R_2$ may only each represent an iodine atom when the 1-aminomethyl-2-aminocyclohexane moiety possess the trans (d) configuration.

The platinum complexes of formula [I] exhibit anti-tumour activity and are potentially of interest as medicaments.

Platinum complexes of the present invention in which

i) $R_1$ and $R_2$ each represent a bromine atom;

ii) $R_1$ and $R_2$ each represent a chlorine atom and the 1-aminomethyl-2-aminocyclohexane moiety is of the trans (d) configuration; and

iii) $R_1$ is $SO_4$ and $R_2$ is $H_2O$ and the 1-aminomethyl-2-aminocyclohexane moiety is of the trans (d) configuration

all possess especially high therapeutic indices and are thus of particular interest for use as medicaments.

The platinum complexes of the present invention may, for example, be produced by any of the following processes, which processes constitute further features of the invention:—

a) for the preparation of a platinum complex as hereinbefore defined (wherein $R_1$ and $R_2$, which are the same, each represents a chlorine, bromine or iodine atom), the reaction of a compound of the formula:—

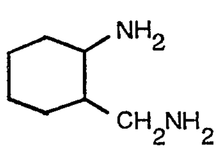

II

with a compound of the formula

$$M^{n\oplus}[PtX_4]^{\ominus}_n \qquad III$$

(wherein X represents a chlorine, bromine or iodine atom, M represents a stabilising metal cation and n is the charge on the cation).

A compound of formula III is preferably used in which X represents chlorine. The stabilising metal cation is preferably an alkali metal or an alkaline earth metal cation e.g. a sodium or potassium cation.

b) for the preparation of a platinum complex as hereinbefore defined (wherein $R_1$ and $R_2$, which are the same, each represent a group $NO_3$), the reaction of a platinum complex of the formula:—

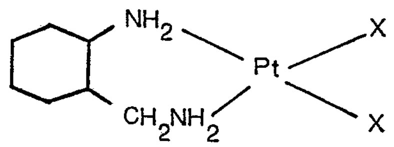

IV

(wherein X represents a halogen atom) with a compound of the formula $B(NO_3)_n$ (in which n represents the charge on B and B is a cation whose halide is insoluble in water) whereby a platinum complex of the formula:—

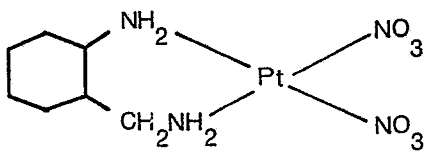

V

is obtained.

The cation B is preferably a silver cation

c) for the preparation of a platinum complex as hereinbefore defined (wherein $R_1$ and $R_2$, which are the same, each represent a bromine or iodine atom or $R_1$ and $R_2$ together form an oxalate, malonate or glucuronate moiety), the reaction of a compound of formula V (as hereinbefore defined) with a compound of the formula:—

(VII) $MX_n$ or $M_2(R_1R_2)_n$ (VIII)

(wherein X represents a bromide or iodide anion, M represents a stabilising metal cation, n represents the charge on the cation and $R_1$ and $R_2$ are as hereinbefore defined) whereby a platinum complex of formula I is obtained.

A compound of formula VII or formula VIII is preferably used in which M represents an alkali metal or alkaline earth metal cation e.g. a potassium or sodium cation.

d) for the preparation of a platinum complex as hereinbefore defined (wherein $R_1$ is $SO_4$ and $R_2$ is $H_2O$), the reaction of a compound of formula IV with a compound of the formula:—

$$B_2(SO_4)_n$$

(wherein B and n are as hereinbefore defined) whereby a compound of the formula:

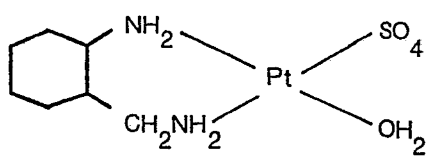

VI

is obtained.

The cation B is preferably a heavy metal cation e.g. a silver cation.

The 1-aminomethyl-2-aminocyclohexane is conveniently prepared as follows:

Racemic (Rac) modification of *trans* (or *cis*)-cyclohexane-1,2-dicarboxylic acid

Step I — Introduction of amino group

Rac-*trans* (or *cis*)-2-aminocyclohexanecarboxylic acid

Step II — Conversion into ester

Rac-*trans* (or *cis*)-2-aminocyclohexanecarboxylic ethyl ester

Step III — Conversion into hydrazide

Rac-*trans* (or *cis*)-2-aminocyclohexanecarbohydrazide

Step IV — Conversion into aminomethyl-compound

Rac-*trans* (or *cis*)-1-aminomethyl-2-aminocyclohexane
[hereinafter referred to as rac-*trans* (or *cis*)-aamch]

Step V — Optical resolution

Trans-d-aamch diastereomer and trans-l-aamch diastereomer
[or cis-d-aamch diastereomer and cis-l-aamch diastereomer]

Step VI — Decomposition of diastereomer

| | | |
|---|---|---|
| Trans-l-aamch | (absolute configuration | : 1R, 2S) |
| Trans-d-aamch | ( ,, | : 2S, 2R) |
| Cis-l-aamch | ( ,, | : 1R, 2R) |
| Cis-d-aamch | ( ,, | : 1S, 2S) |

The above-mentioned steps may, for example, be effected as follows:—

[Step I]

A racemic modification of *trans* (or *cis*)-cyclohexane-1,2-dicarboxylic acid is dissolved in a suitable solvent such as, for example, chloroform, added with an acid and the reaction is carried out to introduce an amino group by using a suitable agent such as, for example, sodium azide. After completion of the reaction, the solvent is removed from the reaction mixture which is then added with a neutralizing agent. After the neutralizing, the solution is filtered and the filtrate is concentrated under reduced pressure to dryness, and if desired, recrystallized from ethanol. As the starting material, one of the market grade may be used.

[Step II]

The racemic modification of *trans* (or *cis*)-2-aminocyclohexanecarboxylic acid prepared by Step I is dissolved in a suitable solvent such as, for example, ethanol. The solution is saturated with hydrogen chloride and at the same time the refluxing ethanol is removed. After this, water is added to the solution which is neutralized with a neutralizing agent and is extracted with ether. Ether is distilled off from the extract to obtain the desired product.

[Step III]

Hydrazine hydrate is added to the *trans* (or *cis*)-2-aminocyclohexanecarboxylic acid prepared by Step II. The solution is refluxed to complete the reaction, and the reaction mixture is concentrated to

dryness under reduced pressure to obtain the desired product which may, if desired, be washed with an organic solvent.

[Step IV]

The *trans* (or *cis*)-2-aminocyclohexanecarbohydrazide is dissolved in a suitable solvent such as, for example, benzene, ether and tetrahydrofuran. The solution is added with a reducing agent such as, for example, lithium aluminium hydride and refluxed for 4 to 7 days. After completion of the reaction, the excessive lithium aluminium hydride is decomposed and the solvent is distilled off, followed by concentration to dryness under reduced pressure to obtain the desired product.

[Step V]

Equimolar amounts of rac-*trans* or *cis*)-aamch prepared by Step IV and (+) [or (−)]-dibenzoyltartaric acid are suspended in water and stirred. After completion of the reaction, the resultant diastereomer is dissolved in a solvent of water/methanol (1:1 volume by volume) and allowed to stand for a night to obtain the needles of the desired product.

[Step VI]

Each isomeric diastereomer prepared by Step V is suspended or dissolved in water and is then treated with conc. hydrochloric acid. The reaction solution is filtered to give a filtrate which is then neutralized to liberate the desired product. This product is, if desired, extracted with an organic solvent and the solvent is evaporated off to obtain the desired product i.e. *trans*-d, *trans*-l, *cis*-d or *cis*-l-aamch.

[Step VII]

The production of platinum complexes from the diamines prepared by Step VI may be carried out in a similar manner to that described, for example, in Journal of Pharmaceutical Sciences, vol. 65, *315—328* (1976) by the following procedures which are as described above:—

1) $PtBr_2$(aamch), $PtI_2$(aamch) and especially $PtCl_2$(aamch) may for example be produced by the reaction of $K_2PtX_4$ (wherein X is chlorine, bromine or iodine) with aamch.

2) The $PtBr_2$(aamch), $PtI_2$(aamch) and $PtCl_2$(aamch) prepared by (1) may for example be reacted with $AgNO_3$ and then filtered to obtain a filtrate. This filtrate is concentrated to dryness to obtain $Pt(NO_3)_2$(aamch).

3) $Pt(NO_3)_2$(aamch) may, for example, be dissolved in water and then treated with KI, KBr, potassium oxalate, sodium malonate or sodium glucuronate to effect the reaction, resulting in $PtI_2$(aamch), $PtBr_2$(aamch), Pt(oxalate)(aamch), Pt(malonate)(aamch) or Pt(glucuronate)(aamch) respectively.

4) $PtCl_2$(aamch) may for example be treated with $AgSO_4$ to carry out the reaction. The resultant AgCl is removed by filtration, and the filtrate is evaporated off to obtain $Pt(OH_2)(SO_4)$-(aamch).

These reactions may for example be effected in the presence of water, if desired, at an elevated temperature and/or in darkness and may be completed in 3 to 48 hours to give precipitates coloured usually in white to yellow. The resultant precipitates may be recrystallized from 0.1N hydrochloric acid to obtain the desired products in the form of crystals.

According to a further feature of the present invention there is provided a pharmaceutical composition comprising as active ingredient at least one platinum complex as hereinbefore defined in association with a pharmaceutical carrier or excipient.

The compositions may be presented in a form suitable for oral, rectal or parenteral administration. Thus, for example, compositions for oral administration may be solid or liquid and may take the form of granules, tablets, coated tablets, capsules, syrups, emulsions, suspensions or drops, such compositions comprising carriers or excipients conventionally used in the pharmaceutical art. Thus, for example, suitable tabletting excipients include lactose, potato and soluble starches and magnesium stearate.

For parenteral administration, the carrier may be a sterile, parenterally acceptable liquid such as sterile water, or a parenterally acceptable oil, e.g. arachis oil, contained in ampoules. Compositions for rectal administration may take the form of suppositories, the carrier comprising a suppository base.

Advantageously, the compositions may be formulated as dosage units, each unit being adapted to supply a fixed dose of active ingredient. Tablets, coated tablets, capsules, suppositories and ampoules are examples of suitable dosage unit forms.

The present invention also provides platinum complexes of formula I as hereinbefore defined when used as a medicament.

The compounds of the present invention may be prepared by the processes of the examples as hereinafter described which are provided by way of illustration only. The physical characteristics of

complexes of the invention are indicated in the following table. Malonate, glucuronate and oxalate are hereinafter abbreviated as mal, gluc and OX respectively.

TABLE I

W: white, LY: light yellow, Y: yellow.

| No. | Compound | Colour | Elemental Analysis Found H | C | N |
|---|---|---|---|---|---|
| 1 | $PtCl_2$(trans-d-aamch) | W | 4.10 | 21.32 | 7.11 |
| 2 | $PtCl_2$(trans-l-aamch) | W | 4.10 | 21.32 | 7.11 |
| 3 | $PtCl_2$ (cis-d-aamch) | W | 4.10 | 21.32 | 7.11 |
| 4 | $PtCl_2$(cis-l-aamch) | W | 4.10 | 21.32 | 7.11 |
| 5 | $PtBr_2$(trans-d-aamch) | LY | 3.34 | 17.40 | 5.80 |
| 6 | $PtBr_2$(trans-l-aamch) | LY | 3.34 | 17.40 | 5.80 |
| 7 | $PtBr_2$(cis-d-aamch) | LY | 3.34 | 17.40 | 5.80 |
| 8 | $PtBr_2$(cis-l-aamch) | LY | 3.34 | 17.40 | 5.80 |
| 9 | $PtI_2$(trans-d-aamch) | Y | 2.80 | 14.57 | 4.85 |
| 10 | Pt(OX)(trans-d-aamch) | W | 3.93 | 26.28 | 6.81 |
| 11 | Pt(OX)(trans-l-aamch) | W | 3.93 | 26.28 | 6.81 |

TABLE I (Continued)

W: white, LY: light yellow, Y: yellow.

| No. | Compound | Colour | Elemental Analysis Calculated H | C | N |
|---|---|---|---|---|---|
| 1 | $PtCl_2$ (trans-d-aamch) | W | 4.18 | 20.98 | 6.88 |
| 2 | $PtCl_2$ (trans-l-aamch) | W | 3.96 | 21.09 | 6.97 |
| 3 | $PtCl_2$ (cis-d-aamch) | W | 4.14 | 21.71 | 7.20 |
| 4 | $PtCl_2$ (cis-l-aamch) | W | 3.93 | 21.20 | 6.96 |
| 5 | $PtBr_2$ (trans-d-aamch) | LY | 3.45 | 18.13 | 2.97 |
| 6 | $PtBr_2$ (trans-l-aamch) | LY | 3.27 | 17.62 | 5.69 |
| 7 | $PtBr_2$ (cis-d-aamch) | LY | 3.36 | 17.75 | 5.95 |
| 8 | $PtBr_2$ (cis-l-aamch) | LY | 3.28 | 17.60 | 5.74 |
| 9 | $PtI_2$ (trans-d-aamch) | Y | 2.95 | 15.93 | 5.45 |
| 10 | Pt(OX)(trans-d-aamch) | W | 3.84 | 26.50 | 6.89 |
| 11 | Pt(OX)(trans-l-aamch) | W | 3.78 | 25.82 | 6.82 |

TABLE I (Continued)

W: white, LY: light yellow, Y: yellow.

| | | | Elemental Analysis | | |
|---|---|---|---|---|---|
| | | | Found | | |
| No. | Compound | Colour | H | C | N |
| 12 | Pt(OX)(cis-d-aamch) | W | 3.93 | 26.28 | 6.81 |
| 13 | Pt(OX)(cis-l-aamch) | W | 3.93 | 26.28 | 6.41 |
| 14 | Pt($OH_2$ $SO_4$) (trans-d-aamch) | W | 4.16 | 19.22 | 6.41 |
| 15 | Pt($OH_2$)($SO_4$)(trans-l-aamch) | W | 4.16 | 19.22 | 6.41 |
| 16 | Pt($OH_2$)($SO_4$)(cis-d-aamch) | W | 4.16 | 19.22 | 6.41 |
| 17 | Pt($OH_2$)($SO_4$)(cis-l-aamch) | W | 4.16 | 19.22 | 6.41 |
| 18 | Pt($NO_3$)$_2$(cis-d-aamch) | W | 3.61 | 18.79 | 12.53 |
| 19 | Pt($NO_3$)$_2$(cis-l-aamch) | W | 3.61 | 18.79 | 12.53 |
| 20 | Pt($NO_3$)$_2$(trans-d-aamch) | W | 3.61 | 18.79 | 12.53 |
| 21 | Pt($NO_3$)$_2$ (trans-l-aamch) | W | 3.61 | 18.79 | 12.53 |
| 22 | Pt(mal)(trans-d-aamch) | W | 4.27 | 28.23 | 6.59 |
| 23 | Pt(mal)(trans-l-aamch) | W | 4.27 | 28.23 | 6.59 |
| 24 | Pt(mal)(cis-d-aamch) | W | 4.27 | 28.23 | 6.59 |
| 25 | Pt(mal)(cis-l-aamch) | W | 4.27 | 28.23 | 6.59 |
| 26 | Pt(gluc)(trans-d-aamch) | LY | | | |
| 27 | Pt(gluc)(trans-l-aamch) | LY | | | |
| 28 | Pt(gluc)(cis-d-aamch) | LY | | | |
| 29 | Pt(gluc)(cis-l-aamch) | LY | | | |

TABLE I (Continued)

W: white, LY: light yellow, Y: yellow.

| No. | Compound | Colour | Elemental Analysis Calculated H | C | N |
|---|---|---|---|---|---|
| 12 | Pt(OX)(cis-d-aamch) | W | 3.32 | 26.12 | 7.00 |
| 13 | Pt(OX)(cis-l-aamch) | W | 3.90 | 26.02 | 6.84 |
| 14 | $Pt(OH_2)(SO_4)$(trans-d-aamch) | W | | | |
| 15 | $Pt(OH_2)(SO_4)$(trans-l-aamch) | W | 4.14 | 19.15 | 6.10 |
| 16 | $Pt(OH_2)(SO_4)$(cis-d-aamch) | W | 4.00 | 19.35 | 6.08 |
| 17 | $Pt(OH_2)(SO_4)$(cis-l-aamch) | W | 4.22 | 19.25 | 6.61 |
| 18 | $Pt(NO_3)_2$(cis-d-aamch) | W | 3.40 | 18.76 | 12.44 |
| 19 | $Pt(NO_3)_2$(cis-l-aamch) | W | 3.57 | 18.91 | 12.38 |
| 20 | $Pt(NO_3)_2$(trans-d-aamch) | W | | | |
| 21 | $Pt(NO_3)_2$(trans-l-aamch) | W | | | |
| 22 | Pt(mal)(trans-d-aamch) | W | 4.08 | 28.05 | 6.62 |
| 23 | Pt(mal)(trans-l-aamch) | W | | | |
| 24 | Pt(mal)(cis-d-aamch) | W | | | |
| 25 | Pt(mal)(cis-l-aamch) | W | | | |
| 26 | Pt(gluc)(trans-d-aamch) | LY | | | |
| 27 | Pt(gluc)(trans-l-aamch) | LY | | | |
| 28 | Pt(gluc)(cis-d-aamch) | LY | | | |
| 29 | Pt(gluc)(cis-l-aamch) | LY | | | |

The following experiments were conducted to investigate the anti-tumour activities of the compounds provided by the present invention.

*Experiments*

$CDF_1$ mouse (each group consisting of 6 mice) were used as test animals. A physiological sodium chloride solution containing $10^6$ cells of Leukemia P—388 was administered to each mice intraperitoneally. On the first and fifth days after this, each mouse was intraperitoneally administered with a given amount of the test compound contained in physiological sodium chloride solution to obtain the results shown in Table 2 wherein the effect is indicated by T/C % (the ratio of the median survival days of the test animal to the control mice).

For comparison purpose, PDD i.e. *cis*-dichlorodiammine platinum (II) which is represented by the following formula:

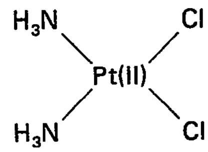

and which is known as being active against tumour cells is treated in a similar manner to that described above to obtain the results shown as Reference in the following tables, from which it it apparent that the platinum complexes provided by the present invention are superior to the known platinum complex with respect to T.I., toxicity and other properties.

TABLE 2

D: Doses (mg/kg)

No.: Compound number

| No. \ D | T/C % | | | | | | | | |
|---|---|---|---|---|---|---|---|---|---|
| | 100 | 50 | 25 | 12.5 | 6.25 | 3.12 | 1.56 | 0.78 | 0.39 |
| 1 | | 61 | 68 | 226 | 174 | 157 | 156 | 138 | 123 |
| 2 | | 62 | 81 | 197 | 177 | 162 | 159 | 140 | 118 |
| 3 | | | | 106 | 243 | 187 | 166 | 147 | 135 |
| 4 | | | | 90 | 240 | 203 | 157 | 138 | 110 |
| 5 | 70 | 194(1) | 188 | 175 | 161 | 145 | 134 | 133 | |
| 6 | 80 | 186 | 178 | 178 | 166 | 164 | 135 | 130 | |
| 7 | 75 | 190 | 196 | 161 | 161 | 147 | 144 | 125 | |
| 8 | 67 | 99 | 181 | 178 | 176 | 147 | 154 | 135 | |
| 9 | 160 | 160 | 143 | 145 | | | | | |
| 10 | | | 171 | 168 | 139 | 131 | 112 | | |
| 11 | | | 83 | 245 | 200 | 151 | 125 | 128 | |
| 12 | | | 90 | 184 | 184 | 131 | 128 | 128 | |
| 13 | | 70 | 193 | 163 | 137 | 146 | 131 | | |
| 14 | | 215 | 196 | 179 | 185 | 166 | 140 | 129 | |
| 15 | | | 80 | 181 | 181 | 151 | 158 | 146 | |
| 16 | | | 59 | 83 | 189 | 174 | 152 | 159 | |
| 17 | | | 59 | 88 | 189 | 178 | 159 | 133 | |
| 18 | | | | 248 | 228 | 197 | | | |
| 19 | | | | 93 | 240 | 220 | | | |
| 26 | | | 170 | 170 | 148 | | | | |
| 27 | | | 200 | 173 | 158 | | | | |
| 28 | | | 180 | 179 | 167 | | | | |
| 29 | | | 208 | 184 | 173 | | | | |
| Ref. | | 0 | 0 | 67 | 121 | 230 | 165 | 134 | 118 |

The toxic dose (T.D), optimal dose (O.D), minimal effective dose (MED), each T/C% corresponding to O.D or MED respectively, and therapeutic index (T.I) of each compound are calculated on the basis of Table 2 and shown in Table 3.

TABLE 3

| No. | T. D ng/$K_2$ | O, D ng/$K_2$ | O, D T/C% | MED ng/$K_2$ | MED T/C% | T. I |
|---|---|---|---|---|---|---|
| 1 | 25 | 125 | 226 | 0.39 | 123 | 32 |
| 2 | 25 | 125 | 197 | 0.78 | 140 | 16 |
| 3 | 25 | 6.25 | 242 | 0.39 | 135 | 16 |
| 4 | 25 | 6.25 | 240 | 0.78 | 138 | 8 |
| 5 | 100 | 50 | 190 (1) | 0.78 | 133 | 64 |
| 6 | 100 | 50 | 186 | 0.78 | 130 | 64 |
| 7 | 100 | 25 | 196 | 0.78 | 125 | 32 |
| 8 | 100 | 25 | 181 | 0.78 | 135 | 32 |
| 9 | ≧200 | 100 | 160 | 12.5 | 145 | 8 |
| 10 | ≧50 | 25 | 171 | 32.1 | 131 | ≧8 |
| 11 | 25 | 125 | 245 | 0.78 | 128 | 16 |
| 12 | 50 | 125 | 184 | 0.78 | 128 | 16 |
| 13 | 50 | 25 | 193 | 1.56 | 131 | 16 |
| 14 | ≧100 | 50 | 215 | 0.78 | 129 | 64 |
| 15 | 75 | 12.5 | 181 | 0.78 | 146 | 16 |
| 16 | 25 | 6.25 | 189 | ≦0.78 | 159 | ≧8 |
| 17 | 25 | 6.25 | 189 | 0.78 | 133 | 8 |
| Ref. | 12.5 | 3.12 | 230 | 0.78 | 134 | 4 |

With reference to the accompanying drawings, Figs. 1—5 show respectively infrared absorption spectra of $PtCl_2$(*trans*-d-aamch), $PtCl_2$(*cis*-1-aamch), $PtBr_2$(*trans*-d-aamch), $PtBr_2$(*cis*-l-aamch) and $PtI_2$-(*trans*-d-aamch). Fig. 6 shows CD spectrum of $Pt(NO_3)_2$(*trans*-l-aamch). Fig. 7 shows CD spectrum of $Pt(NO_3)_2$(*cis*-l-aamch). Fig. 8 shows electron spectrum of $Pt(OH_2)(SO_4)$(*trans*-l-aamch). Fig. 9 (1) and Fig. 9 (2) show respectively CD spectra of $Pt(OH_2)(SO_4)$(*trans*-l-aamch) and $Pt(OH_2)(SO_4)$(*trans*-d-aamch). Figs. 10 and 11 show respectively electron spectrum and CD spectrum of $Pt(OH_2)(SO_4)$(*cis*-l-aamch).

The following non-limitative examples illustrate the invention.

Example 1

(A) *Trans*-cyclohexane-1,2-dicarboxylic acid (8.105 g; racemic modification) is dissolved in conc. sulfuric acid (25 ml) and chloroform (50 ml) and kept at a temperature of 45°C, to which is added sodium azide (3.6 g) in one hour. After this, the solution is stirred for 90 minutes, mixed with ice water (25 ml) and treated with ether to remove chloroform. Barium carbonate (90 g) is added to the water layer of the solution to effect the neutralization. Barium sulfate is removed from the solution by filtration and the filtrate is concentrated to dryness to result in needle crystals of rac-*trans*-2-aminocyclohexanecarboxylic acid (referred to as A) having the following characteristics. When a

corresponding rac-*cis*-compound is used as starting material, there is obtained the corresponding *cis*-compound i.e. rac-*cis*-aminocyclohexanecarboxylic acid, referred to as B).

Elemental analysis (calculated as $C_7H_{13}NO_2$):

| | H | C | N |
|---|---|---|---|
| Calculated: | 58.7% | 9.15% | 9.8% |
| Found (A): | 58.79% | 9.27% | 9.06% |
| (B): | 58.45% | 9.14% | 9.42% |

Melting point: (A) 273°C (B) 238°C

(B) The thus-obtained rac-*trans*-2-aminocyclohexanecarboxylic acid (1.8 g) is dissolved in ethanol. This solution is saturated with hydrogen chloride and refluxed for 7 hours under conditions for saturation with hydrogen chloride. Ethanol is removed from the solution by distillation, and a small amount of water is added to the reaction solution. Ether is distilled off from the reaction solution which is then evaporated under reduced pressure to obtain ethyl ester of rac-*trans*-2-aminocyclohexanecarboxylic acid having a melting point of 120—122°C/266.6 Pa. When a corresponding *cis*-compound is used as starting material, there is obtained the corresponding *cis*-compound having a melting point of 75°C/266.6 Pa.

(C) The thus-obtained ester (trans form; 1.54 g) is mixed with hydrazine hydrate (20 ml) and the mixture is refluxed for one hour. After completion of the reaction, the reaction solution is concentrated under reduced pressure to dryness, resulting in needle crystals of rac-*trans*-2-aminocyclohexanecarbohydrazide having a melting point of 166—167°C. When an ester of the corresponding *cis*-compound is used as starting material, there is obtained rac-*cis*-2-aminocyclohexanecarbohydrazide.

(D) The thus-obtained *trans*-compound (1.57g) is dissolved in a mixture of benzene (100 ml) and tetrahydrofuran (150 ml), treated with lithium alminium hydride, and the mixture refluxed for 7 days. After completion of the reaction, the reaction solution is treated with a saturated solution of potassium carbonate to decompose the excess of lithium alminium hydride. After refluxing for 30 minutes, the solution is filtered and the filtrate is distilled under reduced pressure to remove excess benzene and tetrahydrofuran, resulting in rac-*trans*-1-aminomethyl-2-aminocyclohexane.

(E) The *cis*-compound obtained by (C) (3.5 g) is dissolved in a mixture of benzene (150 ml) and ether (150 ml). The mixed solution is treated with lithium aluminium hydride (2 g) and ether (50 ml) and refluxed for 7 days. After completion of the reaction, lithium aluminium hydride is decomposed. After this, the reaction solution is filtered and the filtrate is distilled to obtain rac-*cis*-1-aminomethyl-2-aminocyclohexane (melting point: 86°C/666.6 Pa).

(F) Rac-*trans*-aamch thus-obtained (17.6521 g) and (+)-dibenzoyltartaric acid (51.7520 g) in an equimolar amount are suspended in water (100 ml) and stirred to prepare a diastereomer which is washed with water several times and is then dissolved in a solvent of water/ethanol (1:1 v/v) at an elevated temperature. The reaction solution is allowed to stand for a night to give needles of *trans*-1-aamch having a specific rotation of $[\alpha]_D^{23} = -95.2°$ which is recrystallized by using a similar mixed solvent to the above-mentioned to a further purified *trans*-1-aamch-diastereomer having a specific rotation of $[\alpha]_D^{23} = -97.78°$. When the rac-*trans*-aamch and (—)-dibenzoyltartaric acid are used in a similar manner to that described above, it is possible to obtain *trans*-d-aamch-diastereomer having a specific rotation of $[\alpha]_D^{23} = +99.06°$.

There is also obtained *cis*-aamch-diastereomer having a specific rotation of $[\alpha]_D^{23} = +91.2°$ or *cis*-d-aamch-diastereomer having a specific rotation of $[\alpha]_D^{23} = -88.7°$ respectively by using rac-*cis*-aamch in combination with either (—)-dibenzoyltartaric acid or (+)-dibenzoyltartaric acid.

(G) Each isomer of the diastereomers thus-obtained is suspended or dissolved in water and treated with conc. hydrochloric acid to liberate benzoyltartaric acid which is then removed by filtration. With addition of sodium hydroxide, the filtrate is neutralized to liberate diamine. The filtrate is then extracted with ether, and ether is removed by distillation to obtain each of the following isomers:

| | | |
|---|---|---|
| *Trans*-l-aamch | ( absolute configuration | :1R,2S) |
| *Trans*-d-aamch | ( ″ | :1S,2R) |
| *Cis*-l-aamch | ( ″ | :1R,2R) |
| *Cis*-d-aamch | ( ″ | :1S,2S) |

By the use of the isomers thus-obtained, it is possible to obtain the platinum complexes of the present invention in the following manner.

(H) Synthesis of $PtCl_2$(aamch):

Aamch (1.28259 g) and $K_2PtCl_4$ (4.1511 g) are dissolved in water (50 ml) and is allowed to stand for some time to give crystalline precipitates coloured in light yellow to white which represent $PtCl_2$(aamch) corresponding to the aamch used as the starting material. The infrared absorption spectrum of $PtCl_2$(*trans*-d-aamch) shown in Fig. 1 is identical with that of $PtCl_2$(*trans*-l-aamch), and the infrared absorption spectrum of $PtCl_2$(*cis*-l-aamch) shown in Fig. 2 is identical with that of $PtCl_2$(*cis*-d-aamch).

(I) Synthesis of $Pt(NO_3)_2$(aamch), $PtBr_2$(aamch) and $PtI_2$(aamch):

$PtCl_2$(aamch) (3.9424 g) and $AgNO_3$ (3.3974 g) are suspended in water (100 ml) and stirred for a night. The AgCl formed is removed from the solution by filtration and the filtrate is evaporated under reduced pressure to dryness, resulting in $Pt(NO_3)_2$(aamch) which is dissolved in water at an elevated temperature and is then treated with an excessive amount of KI or KBr to obtain a desired product coloured in light yellow corresponding to the starting $PtBr_2$(aamch) or a yellow product corresponding to the starting $PtI_2$(aamch).

The infrared absorption spectra of the thus-obtained $PtBr_2$(*trans*-d-aamch), $PtBr_2$(*cis*-l-aamch) and $PtI_2$(*trans*-d-aamch) shown in Figs. 3—5 respectively are identical with the individual infrared spectra of the corresponding optical antipodes. Fig. 6 shows CD spectrum of $Pt(NO_3)_2$(*trans*-l-aamch) measured at a concentration of 1.788 x $10^{-2}$ mol ($\Delta\varepsilon_{323} = +0.025$, $\Delta\varepsilon_{275} = -0.032$). Fig. 7 shows CD spetrum of $Pt(NO_3)_2$(*cis*-l-aamch) measured at a concentration of 3.721 x $10^{-2}$ mol ($\Delta\varepsilon_{355} = +0.020$).

(J) Synthesis of Pt(OX)(aamch), Pt(mal)(aamch) and Pt(gluc)(aamch):

$Pt(NO_3)_2$(aamch) (4.4736 g) and potassium oxalate (1.8424 g) are dissolved in water (10 ml) at an elevated temperature and allowed to stand at ambient temperature to obtain platy crystals of Pt(OX)(aamch) coloured in white.

$Pt(NO_3)_2$(aamch) (4.4736 g) and sodium malonate (1.6604 g) are dissolved in water (10 ml) at an elevated temperature and the solution is concentrated by heating to make up the amount to 5 ml. By allowing the solution to stand at room temperature for a night, white needles of Pt(mal)(aamch) are obtained.

$Pt(NO_3)_2$(aamch) (1.1184 g) and sodium D-glucuronate (0.63049 g) are dissolved in water (5 ml) at an elevated temperature. The solution is allowed to stand at room temperature for a week and is then concentrated under reduced pressure to obtain Pt(gluc)(aamch).

(K) Synthesis of $Pt(OH_2)(SO_4)$(aamch):

$PtCl_2$(aamch) (3.9424 g) and $AgSO_4$ (3.1183 g) are suspended in water (100 ml) and the solution is stirred for a night. After this, the solution is filtered to remove AgCl and the filtrate is concentrated under reduced pressure, followed by addition of acetone to result in white crystals of $Pt(OH_2)(SO_4)$ (aamch).

Figs. 8 and 9(1) show respectively the electron spectrum of $Pt(OH_2)(SO_4)$(*trans*-1-aamch) [measured at a concentration of 1.163 x $10^{-3}$ mol by using a quartz cell (length of the optical pass: 1 cm)] and its CD spectrum [measure at a concentration of 1.163 x $10^{-2}$ mol; $\Delta\varepsilon_{321} = +0.039$; $\Delta\varepsilon_{272} = -0.030$]. Fig. 9(2) shows the CD spectrum of $Pt(OH_2)(SO_4)$(*trans*-d-aamch) [measured at a concentration of 2.027 x $10^{-2}$ mol; $\Delta\varepsilon_{321} = -0.061$; $\delta\varepsilon_{272} = -0.045$]. Figs. 10 and 11 show respectively the electron spectrum of $Pt(OH_2)(SO_4)$(*cis*-1-aamch) [measured at a concentration of 7.86 x $10^{-4}$ mol by using a quartz cell (length of the optical pass: 1 cm)] and its CD spectrum [measured at a concentration of 7.86 x $10^{-3}$ mol; $\Delta\varepsilon_{340} = +0.021$; $\Delta\varepsilon_{265} = +0.068$).

**Claims**

1. A platinum complex of the general formula:

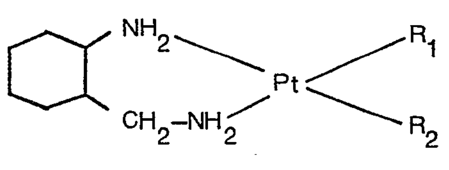

(wherein

a) $R_1$ and $R_2$ are the same and each represents a chlorine, bromine or iodine atom or $NO_3$;

or

b) $R_1$ is $SO_4$ and $R_2$ is $H_2O$;

or

c) $R_1$ and $R_2$ together represent

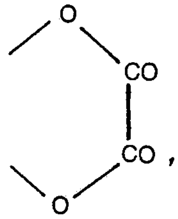 , 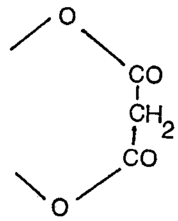

or a glucuronate moiety), the configuration of the 1-aminomethyl-2-aminocyclohexane being selected from *trans*-l, *trans*-d, *cis*-l and *cis*-d, with the proviso that $R_1$ and $R_2$ may only each represent an iodine atom when the 1-aminomethyl-2-aminocyclohexane moiety possesses the trans (d) configuration.

2. A complex as claimed in claim 1 wherein $R_1$ and $R_2$ each represent a bromine atom.

3. A complex as claimed in claim 1 wherein $R_1$ and $R_2$ each represents a chlorine atom and the 1-aminomethyl-2-aminocyclohexane moiety is of the trans (d) configuration.

4. A complex as claimed in claim 1 wherein $R_1$ is $SO_4$ and $R_2$ is $H_2O$, the 1-aminomethyl-2-aminocyclohexane moiety being of the trans (d) configuration.

5. A process for the preparation of a platinum complex as defined in claim 1 (wherein $R_1$ and $R_2$, which are the same, each represents a chlorine, bromine or iodine atom) which comprises reacting a compound of the formula:—

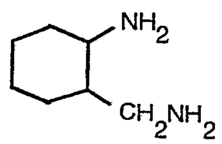

II

with a compound of the formula:—

$$M^{n\oplus}[PtX_4]^{\ominus}_{n} \qquad III$$

(wherein X represents a chlorine, bromine or iodine atom, M represents a stabilising metal cation and n is the charge on the cation).

6. A process for the preparation of a platinum complex as defined in claim 1 (wherein $R_1$ and $R_2$, which are the same, each represent a group $NO_3$), which comprises reacting a platinum complex of the formula:—

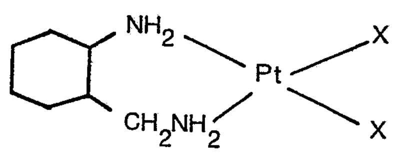

IV

(wherein X represents a halogen atom) with a compound of the formula $B(NO_3)_n$ (in which n represents the charge on B and B is a cation whose halide is insoluble in water) whereby a platinum complex of the formula:—

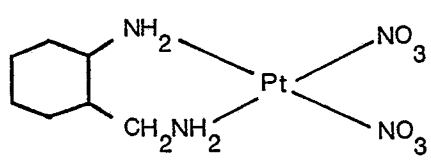

V

is obtained).

7. A process for the preparation of a platinum complex as defined in claim 1 (wherein $R_1$ and $R_2$, which are the same, each represent a bromine or iodine atom or $R_1$ and $R_2$ together form an oxalate, malonate or glucuronate moiety) which process comprises reacting a compound of formula V (as defined in claim 6) with a compound of the formula:—

(VII) $MX_n$ or $M_2(R_1R_2)_n$ (VIII)

(wherein X represents a bromide or iodide anion, M represents a stabilising metal cation, n represents the charge on the cation and $R_1$ and $R_2$ are as defined in claim 1), whereby a platinum complex of formula I is obtained.

8. A process for the preparation of a platinum complex as defined in claim 1 (wherein $R_1$ is $SO_4$ and $R_2$ is $H_2O$) which process comprises reacting a compound of formula IV with a compound of the formula:—

$$B_2(SO_4)_n$$

(wherein B and n are as defined in claim 6) whereby a compound of the formula:—

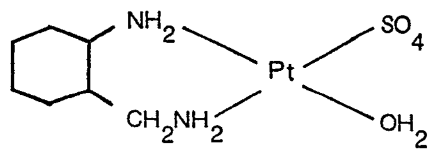

VI

is obtained.

9. A process as claimed in claim 5 or claim 7 wherein a compound III, VII or VIII is used in which M represents an alkali metal.

10. A process as claimed in claim 9 wherein M represents sodium or potassium.

11. A process as claimed in claim 5 or claim 6 wherein X represents chlorine.

12. A process as claimed in claim 6 or claim 8 wherein B represents a silver cation.

13. A pharmaceutical composition comprising as active ingredient at least one platinum complex as defined in claim 1 in association with a pharmaceutical carrier or excipient.

14. A platinum complex as defined in claim 1 for use as a medicament.

**Patentansprüche**

1. Platinkomplex der allgemeinen Formel

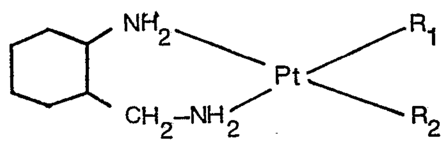

I

(in der

a) $R_1$ und $R_2$ identisch sind und jeweils ein Chlor-, Brom- oder Jodatom oder $NO_3$ bedeuten;

oder

b) $R_1$ $SO_4$ und $R_2$ $H_2O$ ist;

oder

c) $R_1$ und $R_2$ zusammen

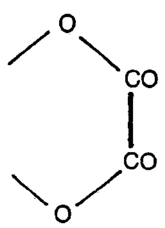 , 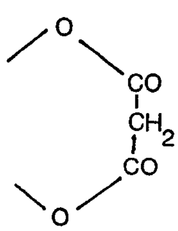

oder einen Glucuronat-Bestandteil bedeuten), wobei die Konfiguration des 1-Aminomethyl-2-amino-cyclohexans aus *trans*-l, *trans*-d, *cis*-l und *cis*-d ausgewählt ist, und zwar mit der Einschränkung, daß $R_1$ und $R_2$ jeweils nur ein Jodatom bedeuten, wenn der 1-Aminomethyl-2-aminocyclohexan-Bestandteil die trans (d)-Konfiguration aufweist.

2. Komplex nach Anspruch 1, wobei $R_1$ und $R_2$ jeweils ein Bromatom bedeuten.

3. Komplex nach Anspruch 1, wobei $R_1$ und $R_2$ jeweils ein Chloratom bedeuten und der 1-Aminomethyl-2-aminocyclohexan-Bestandteil die trans (d)-Konfiguration aufweist.

4. Komplex nach Anspruch 1, wobei $R_1$ $SO_4$ und $R_2$ $H_2O$ ist und der 1-Aminomethyl-2-amino-cyclohexan-Bestandteil die trans (d)-Konfiguration aufweist.

5. Verfahren zur Herstellung eines Platinkomplexes nach Anspruch 1 (wobei $R_1$ und $R_2$ identisch sind und jeweils ein Chlor-, Brom- oder Jodatom bedeuten), welches eine Reaktion einer Verbindung der Formel

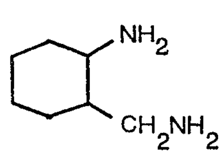

II

mit einer Verbindung der Formel

$$M^{n\oplus}[PtX_4]^{\ominus}_n \qquad III$$

(in der X ein Chlor-, Brom- oder Jodatom, M ein stabilisierendes Metallkation und n die Ladung des Kations bedeuten) beinhaltet.

6. Verfahren zur Herstellung eines Platinkomplexes nach Anspruch 1 (wobei $R_1$ und $R_2$ identisch sind und jeweils eine $NO_3$-Gruppe bedeuten), welches eine Reaktion eines Platinkomplexes der Formel

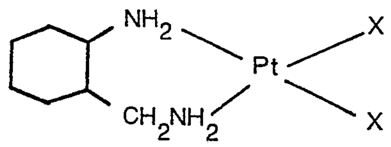

IV

(in der X ein Halogenatom bedeutet) mit einer Verbindung der Formel $B(NO_3)_n$ (in der n die Ladung von B und B ein Kation, dessen Halogenid in Wasser unlöslich ist, bedeuten) beinhaltet, wobei ein Platinkomplex der Formel

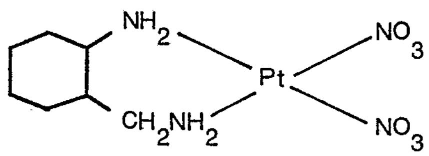

V

erhalten wird.

7. Verfahren zur Herstellung eines Platinkomplexes nach Anspruch 1 (wobei $R_1$ und $R_2$ identisch sind und jeweils ein Brom- oder Jodatom bedeuten oder $R_1$ und $R_2$ zusammen einen Oxalat-, Malonat- oder Glucuronat-Bestandteil bilden), welches eine Reaktion einer Verbindung der Formel V (nach Anspruch 6) mit einer Verbindung der Formel

(VII) $MX_n$ oder $M_2(R_1R_2)_n$ (VIII)

(in denen X ein Brom- oder Jodanion, M ein stabilisierendes Metallkation und n die Ladung des Kations bedeuten und $R_1$ und $R_2$ die im Anspruch 1 angegebene Bedeutung haben) beinhaltet, wobei ein Platinkomplex der Formel I erhalten wird.

8. Verfahren zur Herstellung eines Platinkomplexes nach Anspruch 1 (wobei $R_1$ $SO_4$ und $R_2$ $H_2O$ ist), welches eine Reaktion einer Verbindung der Formel IV mit einer Verbindung der Formel $B_2(SO_4)_n$ (in der B und n die im Anspruch 6 angegebene Bedeutung haben) beinhaltet, wobei eine Verbindung der Formel

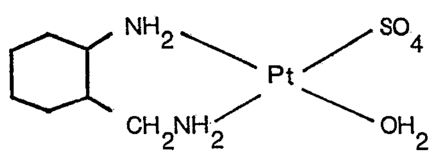

VI

erhalten wird.

9. Verfharen nach Anspruch 5 oder 7, wobei eine Verbindung der Formel III, VII oder VIII, in der M ein Alkalimetall bedeutet, eingesetzt wird.

10. Verfahren nach Anspruch 9, wobei M Natrium oder Kalium bedeutet.

11. Verfahren nach Anspruch 5 oder 6, wobei X Chlor bedeutet.

12. Verfahren nach Anspruch 6 oder 8, wobei B ein Silberkation bedeutet.

13. Pharmazeutische Zusammensetzung, die als aktiven Bestandteil wenigstens einen Platinkomplex nach Anspruch 1 in Verbindung mit einem pharmazeutischen Träger oder Exzipienten enthält.

14. Platinkomplex nach Anspruch 1 zur Verwendung als Medikament.

**Revendications**

1. Complexe de platine de formule générale:

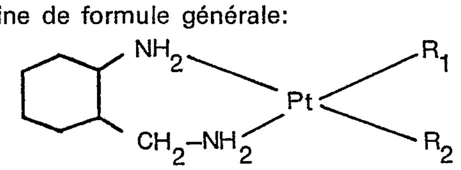

(dans laquelle

a) $R_1$ et $R_2$ sont semblables et représentent chacun un atome de chlore, de brome ou d'iode ou $NO_3$:

ou

b) $R_1$ représente $SO_4$ et $R_2$ représente $H_2O$:

ou

c) $R_1$ et $R_2$ représentent ensemble

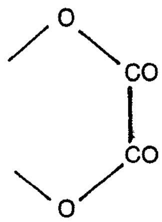 , 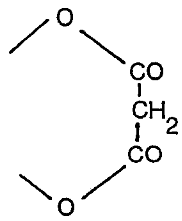

ou un fragment glucuronate), la configuration du 1-aminométhyl-2-aminocyclohexane étant choisie parmi trans-l, trans-d, cis-l et cis-d, sous réserve que $R_1$ et $R_2$ ne peuvent chacun que représenter un atome d'iode lorsque le fragment 1-aminométhyl-2-aminocyclohexane possède la configuration trans (d).

2. Complexe selon la revendication 1 dans lequel $R_1$ et $R_2$ représentent chacun un atome de brome.

3. Complexe selon la revendication 1 dans lequel $R_1$ et $R_2$ représentent chacun un atome de chlore et le fragment 1-aminométhyl-2-aminocyclohexane a la configuration trans (d).

4. Complexe selon la revendication 1 dans lequel $R_1$ représente $SO_4$ et $R_2$ représente $H_2O$, le fragment 1-aminométhyl-2-aminocyclohexane ayant la configuration trans (d).

5. Procédé pour la préparation d'un complexe de platine comme défini dans la revendication 1 (dans lequel $R_1$ et $R_2$ qui sont semblables représentent chacun un atome de chlore, de brome ou d'iode) qui comprend la réaction d'un composé de formule:

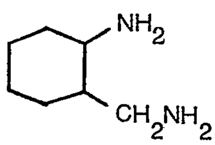

II

avec un composé de formule:

$$M^{n\oplus}[PtX_4]^{\ominus}_n \qquad III$$

(dans laquelle X représente un atome de chlore, de brome ou d'iode, M représente un cation métallique stabilisant et n est la charge du cation).

6. Procédé pour la préparation d'un complexe de platine comme défini dans la revendication 1 (dans lequel $R_1$ et $R_2$ qui sont semblables représentent chacun un groupe $NO_3$) qui comprend la réaction d'un complexe de platine de formule:

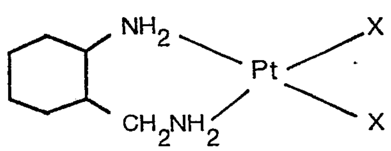

IV

(dans laquelle X représente un atome d'halogène) avec un composé de formule B $(NO_3)_n$ (dans laquelle n représente la charge de B et B est un cation dont l'halogénure est insoluble dans l'eau) pour obtenir un complexe de platine de formule:

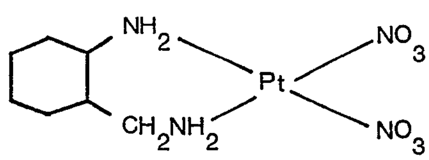

V

7. Procédé pour la préparation d'un complexe de platine comme défini dans la revendication 1 (dans lequel $R_1$ et $R_2$ qui sont semblables représentent chacun un atome de brome ou d'iode ou $R_1$ et $R_2$ forment ensemble un fragment oxalate, malonate ou glucuronate), ce procédé comprenant la réaction d'un composé de formule V (comme défini dans la revendication 6) avec un composé de formules:

(VII) $MX_n$ ou $M_2(R_1R_2)_n$ (VIII)

(dans lequelles X represents un anion bromure ou iodure, M représente un cation métallique stabilisant,

n représente la charge du cation, et $R_1$ et $R_2$ ont la même définition que dans la revendication 1) pour obtenir un complexe de platine de formule I.

8. Procédé pour la préparation d'un complexe de platine comme défini dans la revendication 1 (dans lequel $R_1$ représente $SO_4$ et $R_2$ représente $H_2O$), ce procédé comprenant la réaction d'un composé de formule IV avec un composé de formule: $B_2(SO_4)_n$ (dans laquelle B et n ont la même définition que dans la revendication 6) pour obtenir un composé de formule.

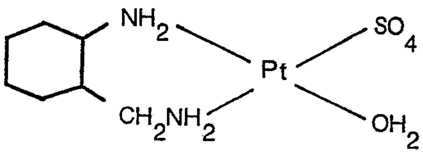

VI

9. Procédé selon l'une des revendications 5 ou 7 dans lequel on utilise un composé III, VII ou VIII dans lequel M représente un métal alcalin.

10. Procédé selon la revendication 9 dans lequel M représente le sodium ou le potassium.

11. Procédé selon l'une des revendications 5 ou 6 dans lequel X représente le chlore.

12. Procédé selon l'une des revendications 6 ou 8 dans lequel B représente un cation argent.

13. Composition pharmaceutique comprenant comme ingrédient actif au moins un complexe de platine comme défini dans la revendication 1 en association avec un véhicule ou excipient pharmaceutiques.

14. Complexe de platine comme défini dans la revendication 1 pour l'emploi comme médicament.

0 008 936

FIG. 1

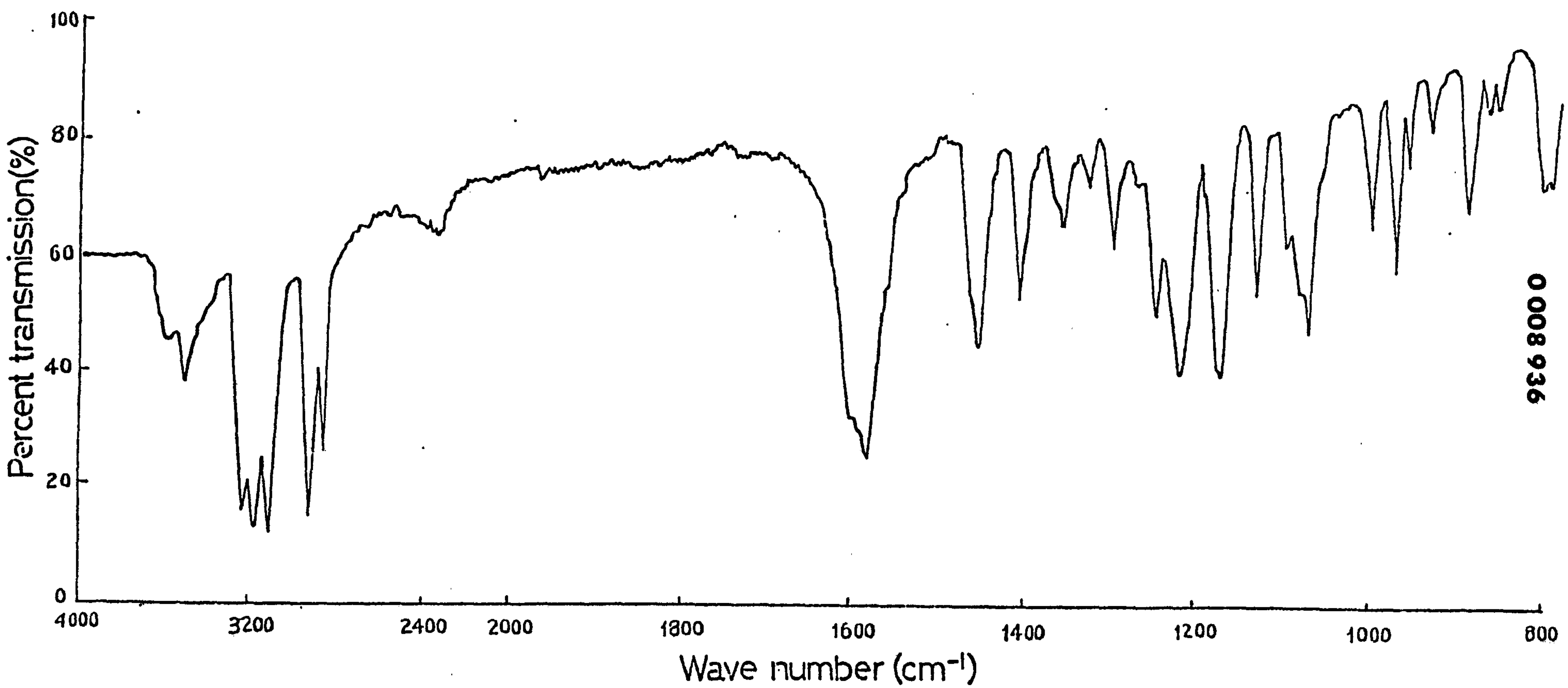

FIG. 2

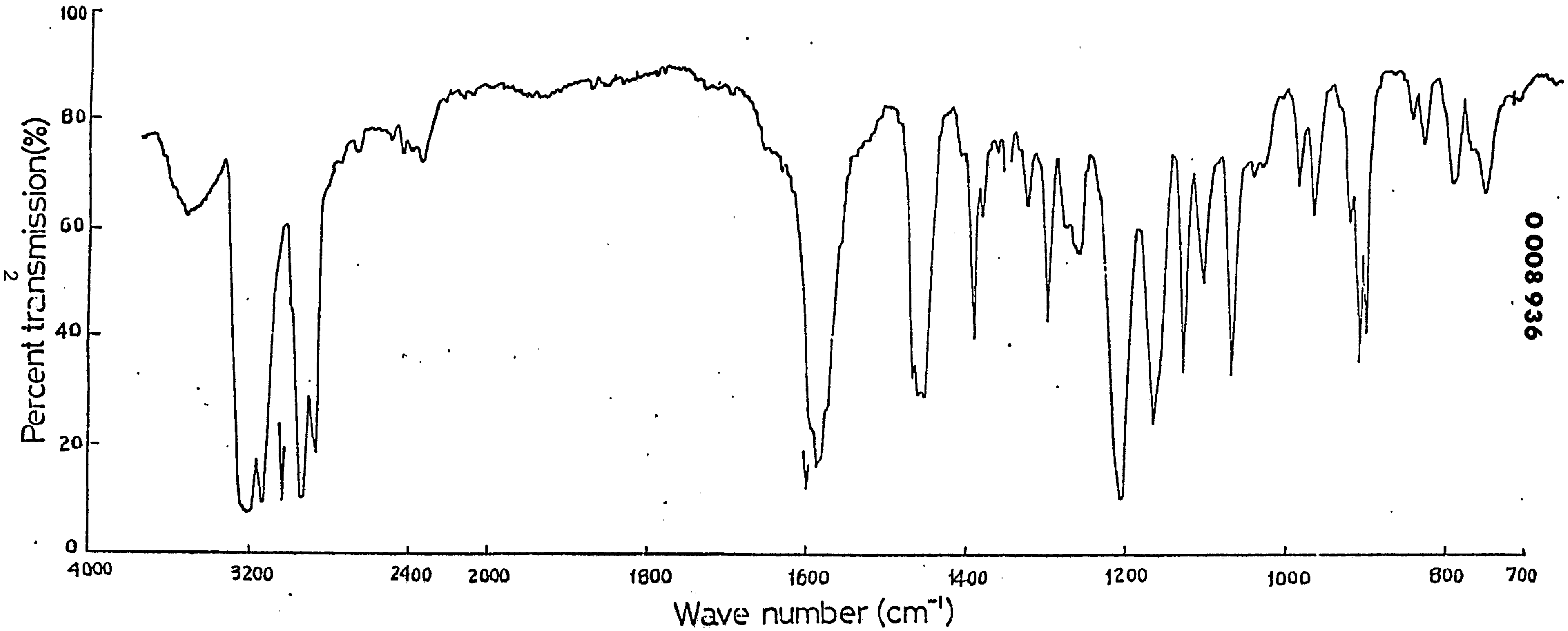
100
80
60
40
20
0
Percent transmission(%)
4000
3200
2400
2000
1800
1600
1400
1200
1000
800
700
Wave number ($cm^{-1}$)

FIG. 3

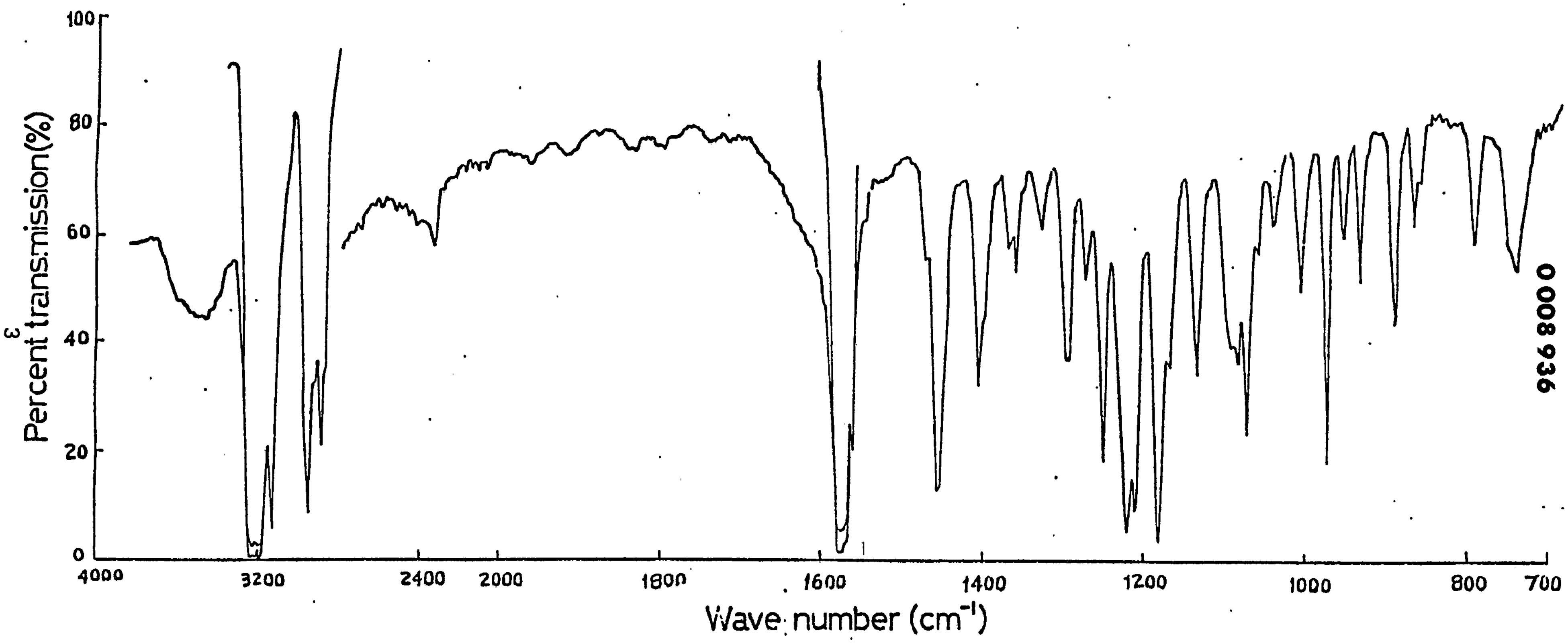
Percent transmission(%)
100
80
60
40
20
0
4000
3200
2400
2000
1800
1600
1400
1200
1000
800
700
Wave number (cm$^{-1}$)

FIG. 4

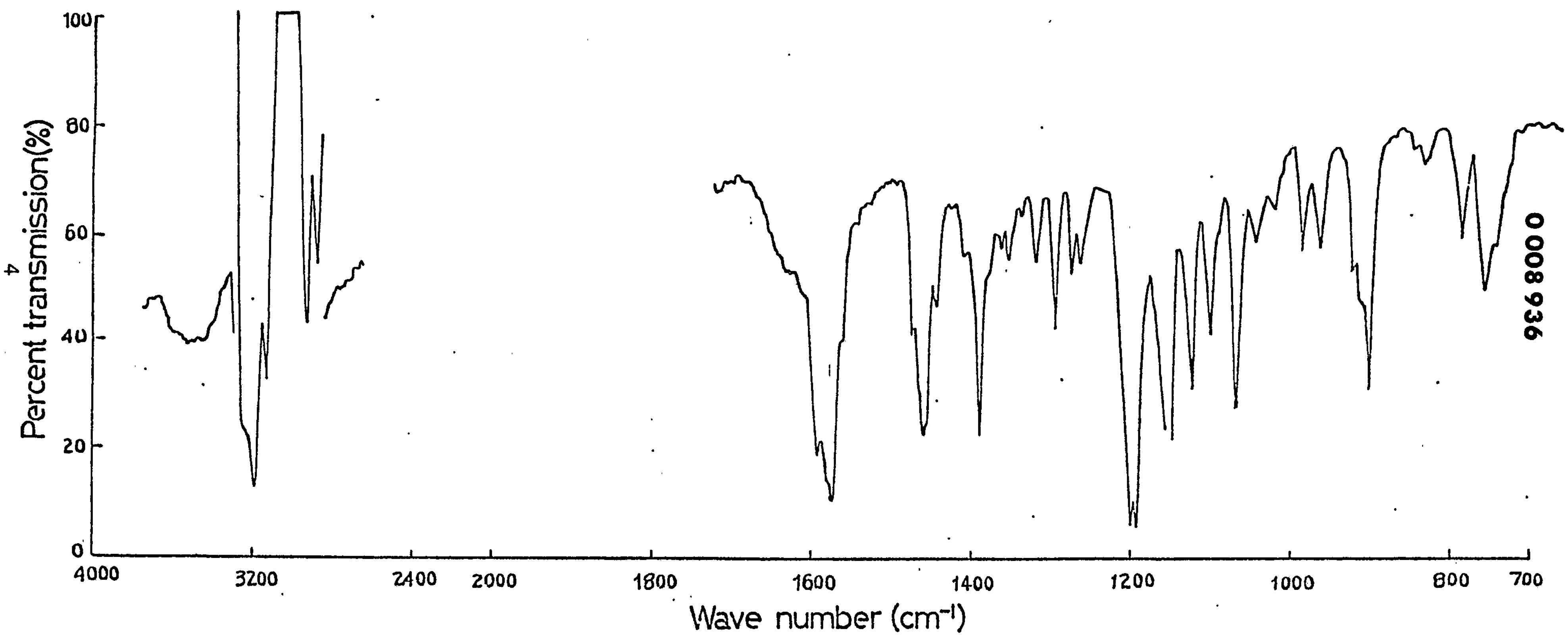
Percent transmission(%)
100
80
60
40
20
0
4000
3200
2400
2000
1800
1600
1400
1200
1000
800
700
Wave number (cm⁻¹)

FIG. 5

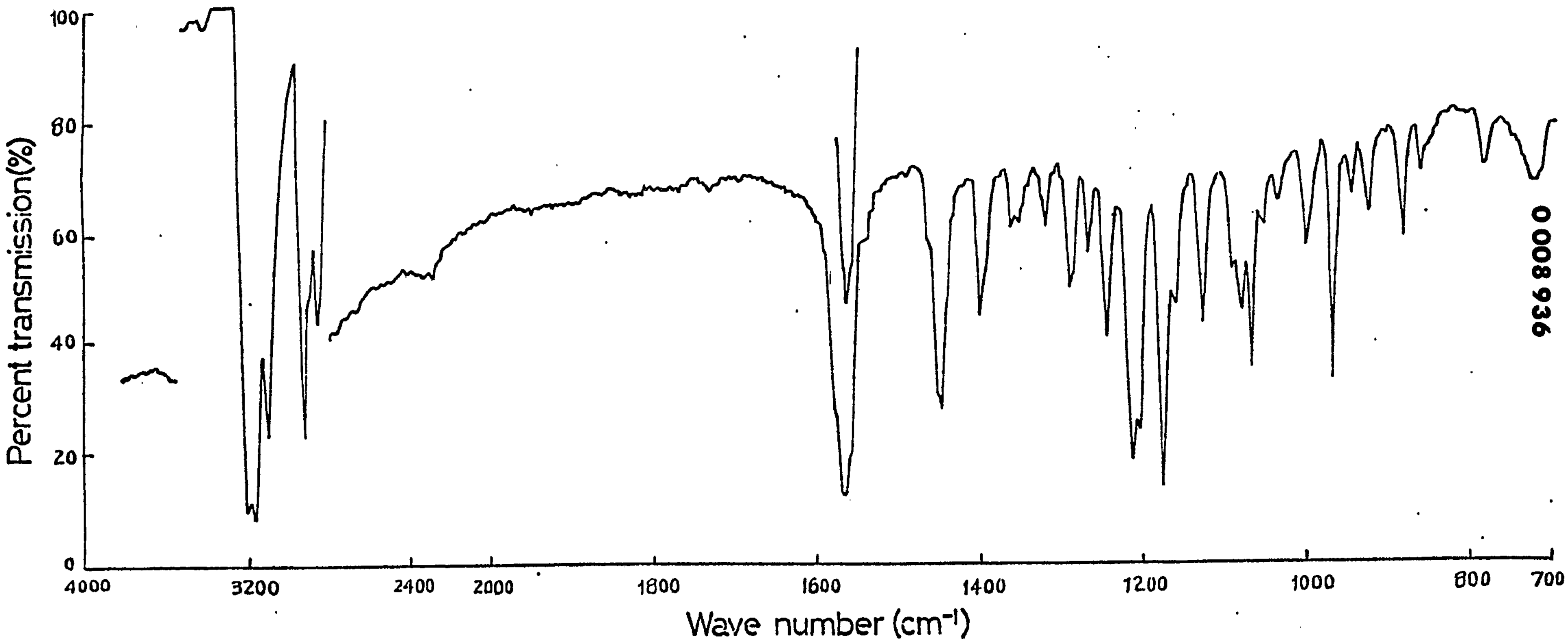
Percent transmission(%)
100
80
60
40
20
0
4000
3200
2400
2000
1800
1600
1400
1200
1000
800
700
Wave number (cm⁻¹)

FIG. 6

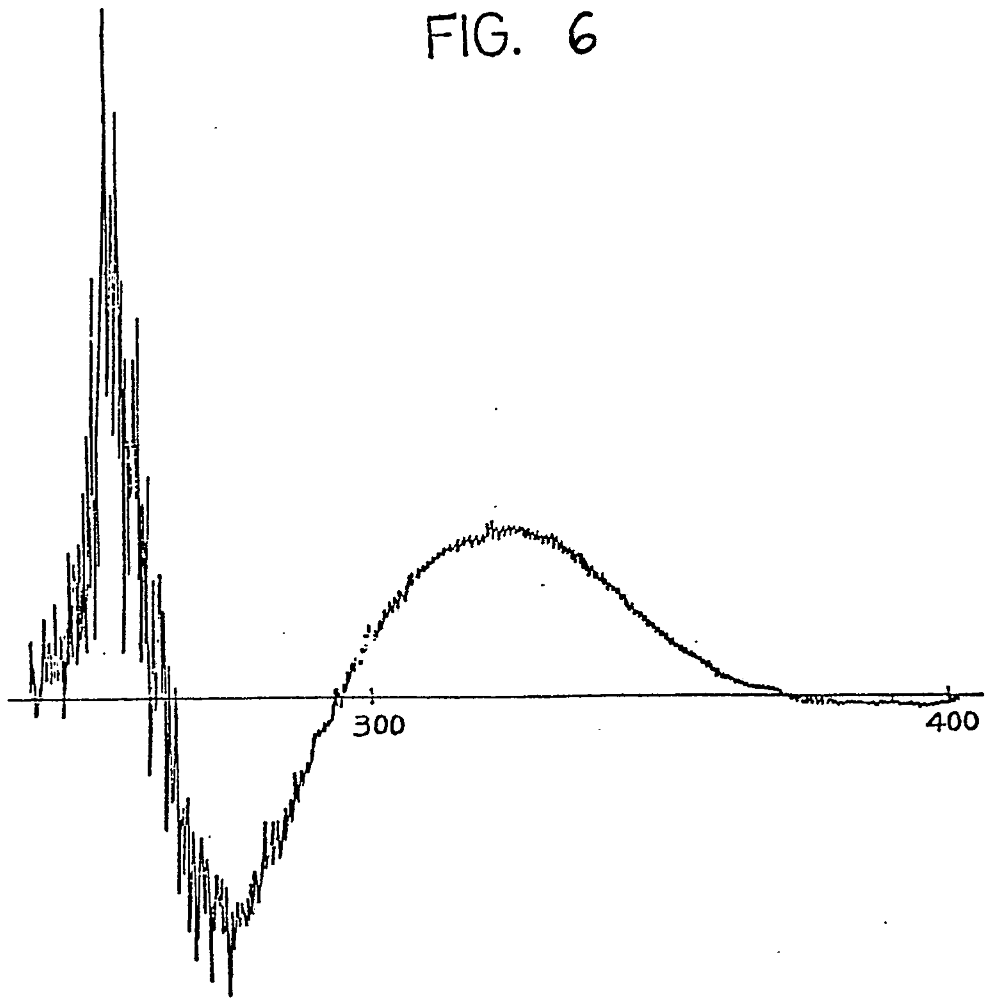


FIG. 7

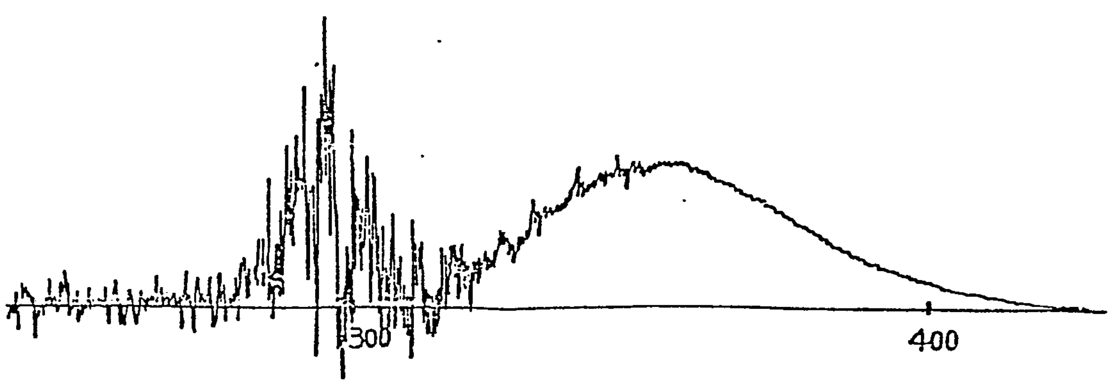

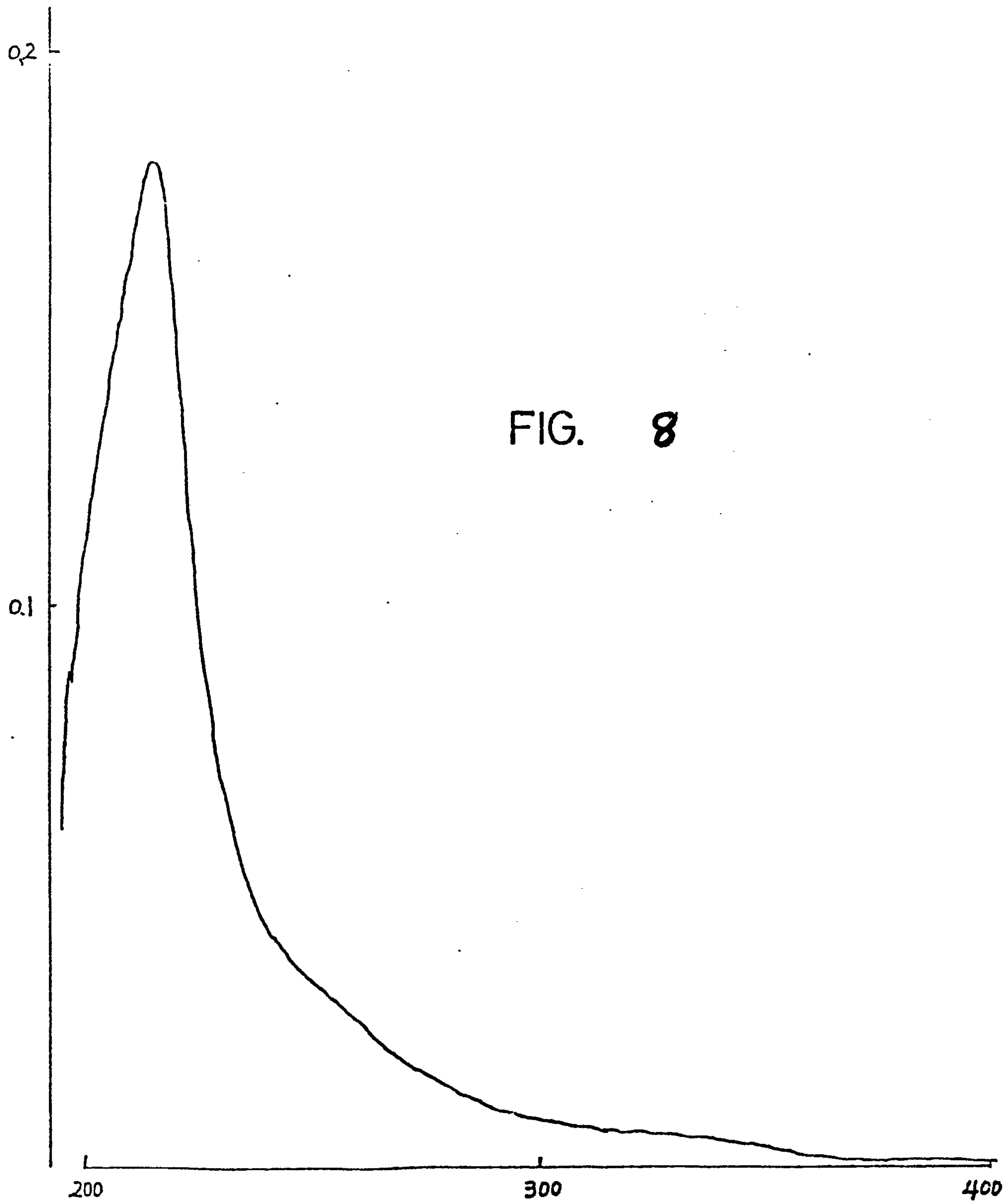


FIG. 8

FIG. 9

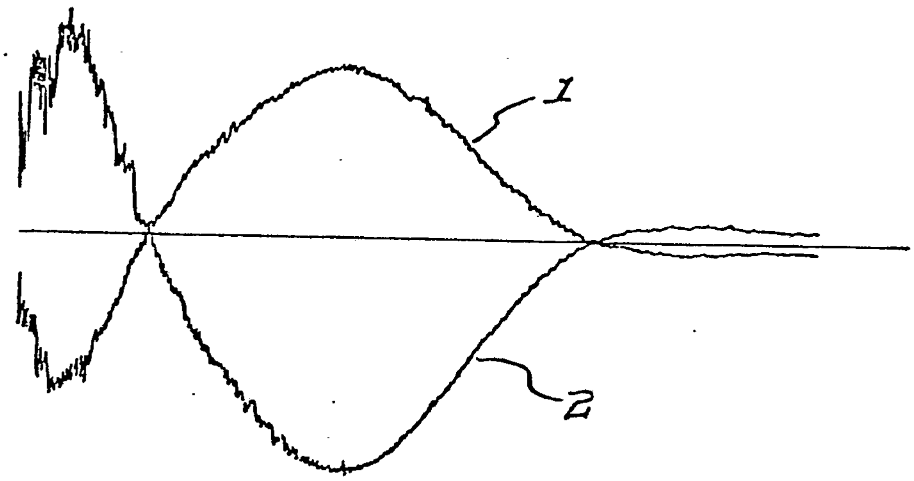


FIG. 11

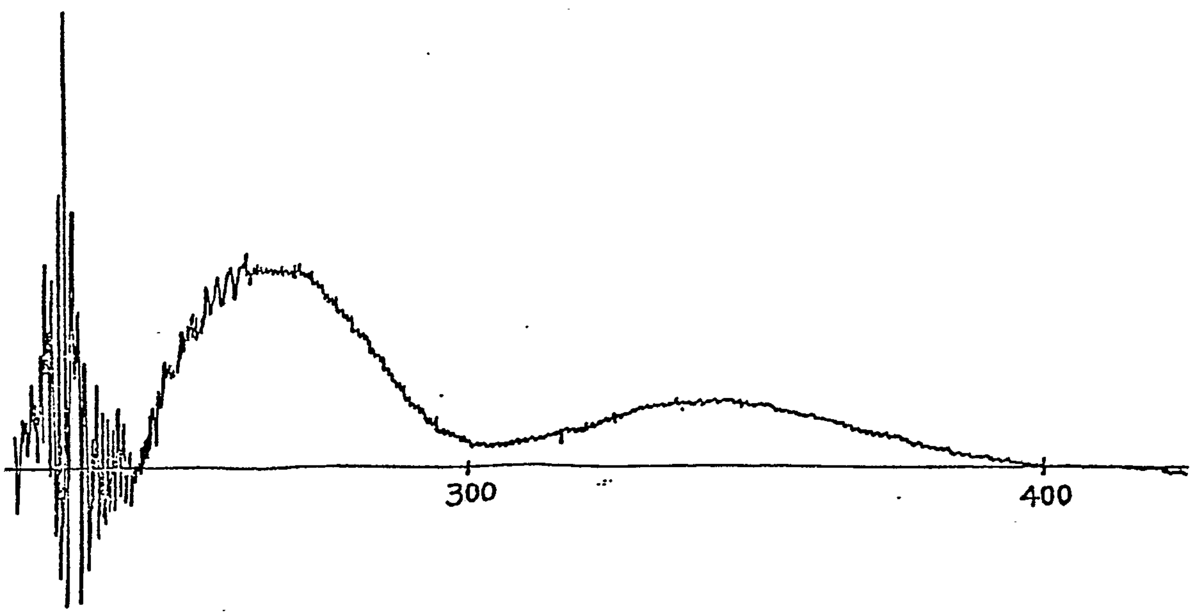

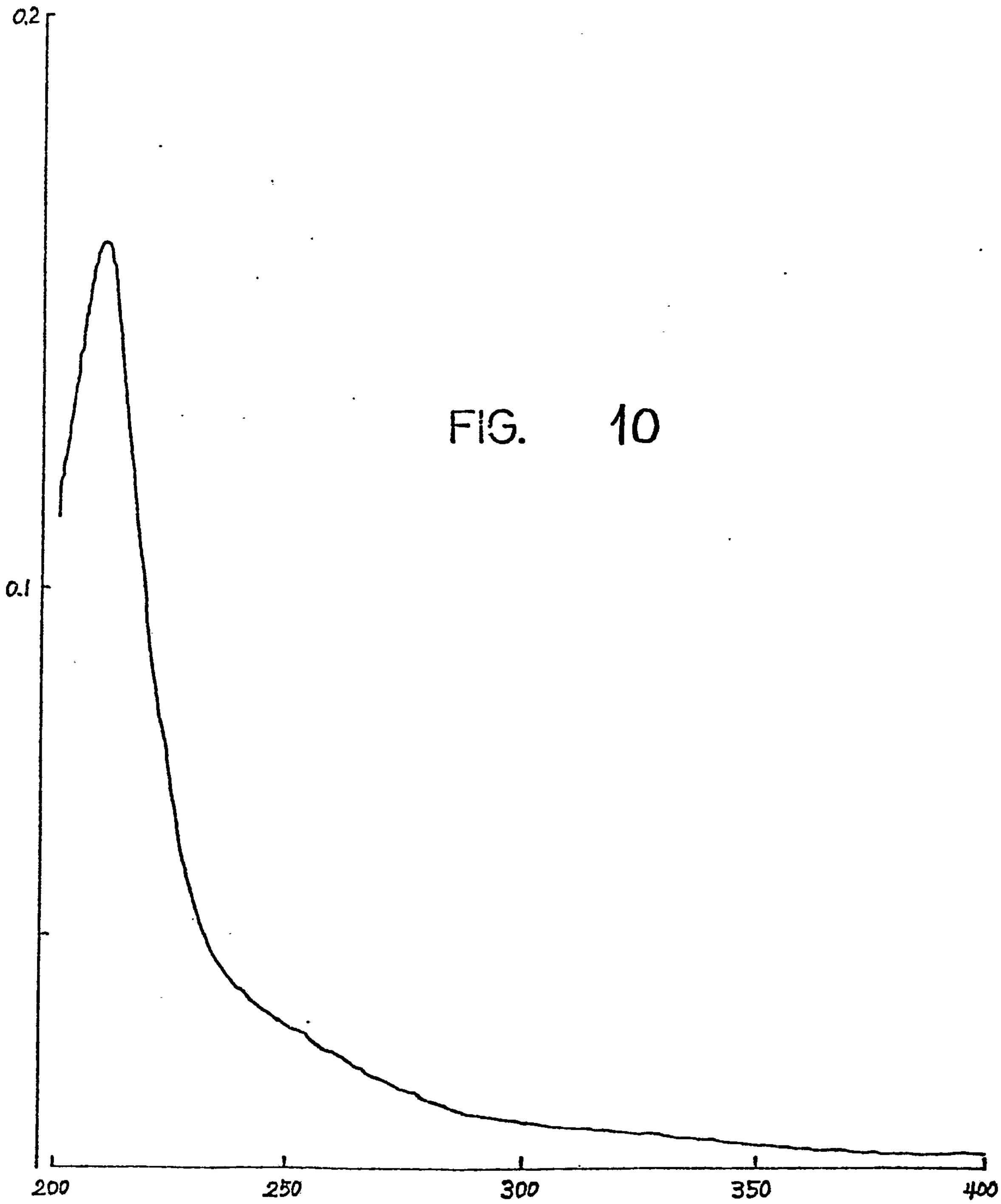


FIG. 10